# DEMANDE DE BREVET EUROPEEN

(11) **EP 1 522 311 A1**
(43) Date de publication de la demande: **13.04.2005**
(21) Numéro de dépôt: 03292505.9
(22) Date de dépôt: 10.10.2003
(51) Int. Cl.: A61K 38/31, A61K 38/16

(54) **Utilisation de la somatostine ou d'un de ses analogues pour préparer un médicament destiné à réguler la réserve folliculaire ovarienne chez la femme non ménopausée**

(71) Demandeur: INSTITUT NATIONAL DE LA SANTE ET DE LA RECHERCHE MEDICALE (INSERM), 75013 Paris (FR); Société De Conseils De Recherches et D'Applications Scientifiques (S.C.R.A.S.), 75781 Paris Cédex 16 (FR)
(72) Inventeur: Gougeon, Alain, 69630 Chaponost (FR); Loumaye, Ernest, 74140 Massongy (FR)
(74) Mandataire: Bourgouin, André

(57) **Abrégé**

L'invention concerne l'utilisation de la somatostatine ou d'un de ses analogues agonistes pour préparer un médicament destiné à réguler la réserve folliculaire ovarienne, et en particulier à diminuer l'épuisement de la réserve folliculaire ovarienne avec le temps, chez la femme non ménopausée, ou celle d'un analogue antagoniste de la somatostatine pour préparer un médicament destiné à accélérer l'entrée en croissance des follicules quiescents chez la femme non ménopausée.

## Description

L'invention concerne l'utilisation de la somatostatine ou d'un de ses analogues agonistes pour préparer un médicament destiné à réguler la réserve folliculaire ovarienne chez la femme non ménopausée, ou celle d'un analogue antagoniste de la somatostatine pour préparer un médicament destiné à accélérer l'entrée en croissance des follicules quiescents chez la femme non ménopausée.

Chez la femme, comme chez tous les mammifères, la fertilité est dépendante de la présence dans les ovaires des gamètes femelles appelés "ovocytes". Dans l'espèce humaine, le capital ovocytaire est constitué une fois pour toutes à la naissance ; le nombre d'ovocytes est alors compris entre 500 000 et 1 million par ovaire. Ces ovocytes sont entourés de quelques cellules de la granulosa ; cet ensemble fonctionnel est appelé follicule ovarien (Gougeon, A., *Endocrine Reviews* (1996), **17**, 121-155). A la naissance, mais aussi tout au long de la vie jusqu'à la ménopause, la majorité des follicules ovariens sont à un stade de repos.

Dès sa constitution, le capital ovocytaire s'amenuisera progressivement : ainsi, il y aura environ 200 000 follicules par ovaire à la puberté, environ 80 000 à 20 ans, environ 30 000 à 30 ans, environ 10 000 à 40 ans, le capital étant pratiquement épuisé vers l'âge de 50 ans (cf. Gougeon, A. et Lefèvre, B., « Folliculogénèse et maturation ovocytaire » dans *Médecine de la reproduction,* 3^{e} édition, Ed. Flammarion, p. 49). L'épuisement du capital ovocytaire correspond cliniquement à la ménopause. Les follicules au repos présents dans l'ovaire à un moment donné constituent la "réserve ovarienne".

Deux mécanismes sont impliqués dans l'épuisement progressif de la réserve ovarienne. Environ 80% des follicules disparaîtront par entrée en apoptose, tandis que les 20% restants entreront en croissance. Ces derniers entreprendront alors un long processus de développement (environ 6 mois) menant une minorité d'entre eux (environ 400 pendant toute la vie) au stade de follicules préovulatoires contenant un ovocyte mature apte à être fécondé (Gougeon, A., *Endocrine Reviews* (1996), **17,** 121-155). La majorité des follicules en croissance disparaissent par apoptose conduisant à leur involution ; l'apoptose les frappe à n'importe quel stade de leur développement.

Le passage du stade follicule quiescent au stade de follicule en croissance est un phénomène continu mais d'intensité variable. En particulier, il s'accélère dans les 10 à 15 années précédant la ménopause, dès l'âge d'environ 38 ans.

Les facteurs stimulant les premières étapes de croissance (à partir du grand follicule primaire) sont relativement bien connus. Ils incluent les gonadotrophines (LH et FSH) mais surtout des facteurs de croissance et des stéroïdes tels que les androgènes. Par contre, les mécanismes contrôlant l'initiation de la croissance folliculaire sont mal connus. Il est bien établi que cette étape de la folliculogénèse n'est pas dépendante des gonadotrophines (LH et FSH) (cf. par exemple Bullun, S. et Adashi, E., *Williams Textbook of Endocrinology,* Tenth Edition (2003), 587-664). Une hormone nommée AMH (Anti-Mullerian hormone) pourrait être impliquée dans le maintien en quiescence des follicules tandis qu'un peptide nommé Kit-Ligand (aussi appelé SCF) pourrait être impliqué dans l'activation des follicules au repos vers la croissance. En outre, un facteur de croissance nommé GDF-9 semble important pour soutenir la croissance une fois enclenchée.

La somatostatine (SST) est un peptide cyclique présent sous deux formes dans l'organisme, une forme contenant 14 acides aminés et une forme contenant 28 acides aminés. L'activité biologique de ces deux formes de SST est semblable. La forme SST-14 est la forme prédominante au niveau du système nerveux central. Elle inhibe la sécrétion de l'hormone de croissance par les cellules somatotropes de l'anté-hypophyse. La forme SST-28 est préférentiellement exprimée au niveau de l'estomac et du pancréas. L'activité biologique de la SST est induite par l'intermédiaire d'une série de récepteurs membranaires couplés à une protéine G, dont 5 sous types ont été caractérisés, à savoir les sous-types SSTR1 à SSTR5 (Reubi, J.C., *Cancer Res.,* **47,** 551-558 ; Resine, T., et coll., *Endocr. Review,* **16,** 427 - 442 ; Lamberts, SW. et coll., *Endocr. Review,* **12,** 450-482).

La présence de SST au niveau de l'ovaire a été démontrée chez plusieurs espèces dont le porc (Mori, T. et coll., *Acta Endocrinol. (Copenh.)* (1984), **106**(2), 254-259), le rat (McNeill, D.L. et coll., *Am. J. Anat.* (1987), **179**(3), 269-76) et la femme (Holst et coll., *Hum. Reprod.* (1994), **9**(8), 1448-1451). Les récepteurs à la SST ont été identifiés dans l'ovaire du rat (Lidor, A. et coll., *Gynecol. Endocrinol.* (1998), **12**(2), 97-101) ainsi que dans l'ovaire humain en particulier les sous types 1, 2A et 5 (Strauss et coll., *Hum. Reprod.* (2003), **18**, Suppl. 1, P-495).

La contribution de la SST dans la physiologie ovarienne a été étudiée par plusieurs auteurs. Chez le rat, l'administration *in vivo* de SST semble réduire le nombre de cellules hypophysaires produisant LH et FSH ainsi que le nombre de follicules pré-ovulatoires dans l'ovaire (Nestorovic et coll., *Histochem. J.* (2001), **33**(11-12), 695-702). *In vitro,* la SST inhibe l'aromatase et la production de progestérone, stimulées par FSH, dans un modèle de cellules de la granulosa (Andreani, C.L. et coll., *Hum. Reprod.* (1995), **10**(8), 1968-1973). Chez le porc, la SST inhibe l'augmentation de l'AMPc induite par LH et la forskoline dans les cellules de la granulosa (Rajkumar, K. et coll., *J. Endocrinol.* (1992), **134**(2), 297-306), et semble inhiber la maturation nucléaire de l'ovocyte préovulatoire (Mori, T. et coll., *Acta Endocrinol. (Copenh.)* (1985), **110**(3), 408-412). Chez la femme, des études *in vitro* sur des cellules de la granulosa issues de follicules pré-ovulatoires suggèrent un rôle inhibiteur direct de la SST sur la synthèse de l'IGF-BP1 et de la progestérone (Holst, N. et coll., *Fertil. Steril.* (1997), **68**(3), 478-482). Chez la femme, *in vivo,* SST est capable de réduire la sécrétion de LH par l'hypophyse, de diminuer la production d'androgènes et les taux sériques de l'IGF-1. Par contre, SST augmente les taux sériques de l'IGF-BP3 (Fulghesu, A.M. et coll., *Fertil. Steril.* (1995), **64**(4), 703-708; Piaditis, G.P. et coll., *Clin. Endocrinol. (Oxf.)* (1996), **45**(5), 595-604). La SST a été co-administrée avec de la FSH lors de traitement d'induction de l'ovulation chez des patientes présentant une infertilité résultant d'un syndrome des ovaires polycystiques. La capacité de la SST à diminuer les taux sériques de LH, et à diminuer les taux sériques d'hormone de croissance et d'IGF-I a été confirmée. Cet effet endocrinien ne s'est pas cependant pas accompagné d'un impact significatif sur la croissance folliculaire induite par l'administration de FSH (Lidor, A. et coll., *Gynecol. Endocrinol.* (1998), **12**(2), 97-101; van der Meer, M. et coll., *Hum. Reprod.* (1998), **13**(6), 1465-1469). En résumé, il a été rapporté à ce jour un effet marginal de la SST sur la sécrétion hypophysaire de LH et sur la production de progestérone par les cellules de la granulosa des follicules pré-ovulatoires.

Les Demanderesses ont maintenant découvert de façon surprenante que la SST et ses analogues ont la capacité d'inhiber le passage des follicules d'un stade quiescent à un stade de croissance et que cette faculté ouvrait à ces composés de nouvelles utilisations thérapeutiques.

L'intérêt de cette découverte réside en premier lieu dans l'opportunité d'utiliser la SST ou un analogue agoniste de la SST pour préparer un médicament destiné à diminuer, voire inhiber l'entrée en croissance des follicules au stade quiescent. En second lieu, l'on pourra aussi utiliser un analogue antagoniste de ce peptide pour préparer un médicament destiné à accélérer l'entrée en croissance des follicules quiescents.

Il existe en effet un ensemble de situations cliniques pour lesquelles il serait médicalement souhaitable pour la patiente de ralentir l'utilisation de la réserve ovarienne afin de différer l'épuisement de cette dernière et donc de préserver la fonction ovarienne et la fertilité. Ces situations sont typiquement, et de manière non exclusive, les patientes à risque de ménopause précoce. Il est en effet bien connu que certaines patientes présentent un épuisement prématuré de leur capital folliculaire. La ménopause survient alors avant l'âge de 40 ans et parfois même avant l'âge de trente ans. Il est souvent possible de prévoir cette ménopause précoce sur la base d'antécédents familiaux, ou d'anomalies génétiques telles que le syndrome de Turner (complet ou partiel). Dans cette situation, l'administration de SST ou d'un de ses analogues agonistes sera préventive et aura pour but de ralentir l'entrée en croissance des follicules quiescents.

Il en est de même pour les patientes présentant une difficulté à concevoir et dont l'âge chronologique ou l'âge biologique de leurs ovaires correspond à la période d'accélération de la mobilisation des follicules quiescents : ralentir cet épuisement du capital folliculaire devrait permettre d'augmenter l'efficacité des traitements et l'opportunité d'obtenir une grossesse.

Une autre situation clinique pouvant bénéficier d'un traitement par la SST ou par un de ses analogues agonistes est la greffe (préférablement autologue) d'ovaire ou de fragments d'ovaire. Dans ce contexte, la reprise de la fonction ovarienne est souvent transitoire et s'accompagne d'une épuisement précoce du nombre de follicules primordiaux (Baird, D.T. et coll., *Endocrinology* (1999), **140**, 462-471). Il a en effet été démontré que, lors de la transplantation, les cellules de la granulosa des follicules en croissance sont plus disposées à entreprendre un phénomène d'apoptose que celle des follicules primordiaux (Liu, J. et coll., *Hum. Reprod.* (2002), **17,** 605-611). De plus, le prélèvement de tissu ovarien et leur fragmentation entraînent une mobilisation massive et rapide des follicules primordiaux vers un stade de follicules primaires tardifs (cf. Wandji S-A, et al., *Hum. Reprod.* (1997), **12,** 1993-2001; cf. également le groupe contrôle de l'exemple de la présente demande).

En dehors des pathologies évoquées précédemment, un ralentissement systématique de l'épuisement de la réserve ovarienne pourrait être envisagé chez la femme ne souffrant d'aucun dysfonctionnement ovarien. Dans les pays industrialisés, l'allongement continu de l'espérance de vie (actuellement environ 83 ans en France) s'accompagne d'un allongement de la période post-ménopausique et des troubles qui y sont associés : cardiopathies, ostéoporose, vieillissement cutané, etc. Des doutes apparaissent quant à l'innocuité à long terme des traitements hormonaux substitutifs de la ménopause. En conséquence, une alternative élégante consisterait à décaler l'âge de survenue de la ménopause. Cela réduirait d'autant la période postménopausique et les risques associés. Ce décalage ne signifierait pas pour autant que la fertilité pourrait être maintenue jusqu'à 60 ans et plus. En effet de nombreux travaux suggèrent que la fonction ovarienne est maintenue tant qu'un nombre minimum de follicules de la réserve est maintenu, et qu'en dépit d'une fonction ovarienne "normale" (taux de stéroïdes peu affectés), les possibilités de grossesse sont extrêmement faibles.

Dans les situations où l'on souhaite ralentir l'utilisation de la réserve ovarienne, on utilisera selon l'invention la somatostatine naturelle (SST14 ou SST28), ou, de préférence, un analogue agoniste de la somatostatine (naturel ou synthétique). L'analogue agoniste de la somatostatine peut être un polypeptide cyclique ou non cyclique, une protéine de fusion ou de recombinaison, une entité chimique non peptidique (i.e. peptidomimétique) ou encore un peptide *« SS-like »* tel que la corticostatine. Les analogues agonistes à utiliser devront présenter une haute affinité pour le récepteur SST et induire une activité fonctionnelle de celui-ci telle que l'inhibition de la sécrétion d'hormone de croissance par des cellules somatotropes de l'hypophyse et/ou l'inhibition de la prolifération in vitro de cellules d'adénome hypophysaire. De préférence, l'analogue agoniste de la somatostatine aura une haute affinité pour l'ensemble des sous types de récepteurs de la SST ou alors une affinité privilégiée pour au moins l'un des sous-types 1, 2, 3, 4 et 5.

Des analogues agonistes de la somatostatine ont été décrits notamment dans la demande de brevet PCT WO 01/00676 ou WO 98/08528 ou encore dans les brevets US 6,387,932, US 6,268,342, US 6,057,338, US 6,025,372.

Parmi les analogues agonistes de la somatostatine utilisables selon l'invention, on peut citer plus particulièrement le lanréotide, l'octréotide, le vapréotide, le SOM 230 (voir structure ci-dessous), le MK-678 (voir structure ci-dessous), le BIM-23190 (peptide de structure N-hydroxyéthylpipérazinyl-acétyl-D-Phe-cyclo[Cys-Tyr-D-Trp-Lys-Abu-Cys]-Thr-NH₂), le BIM-23197 (peptide de structure Hepes-D-Phe-cyclo[Cys-Tyr-D-Trp-Lys-Abu-Cys]-Thr-NH₂ dans laquelle Abu représente l'acide aminobutyrique), le BIM-23268 (peptide de structure cyclo[Cys-Phe-Phe-D-Trp-Lys-Thr-Phe-Cys]-NH₂), le PTR-3173 (voir structure ci-dessous), le TT-232 (de structure D-Phe-cyclo[Cys-D-Trp-Lys-Cys]-Thr-NH₂), et leurs sels pharmaceutiquement acceptables. On préférera tout particulièrement employer le lanréotide, l'octréotide ou un de leurs sels pharmaceutiquement acceptables, et plus particulièrement le lanréotide ou un de ses sels pharmaceutiquement acceptables.

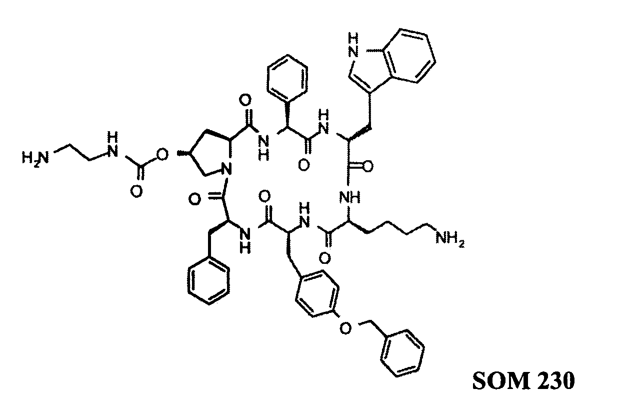

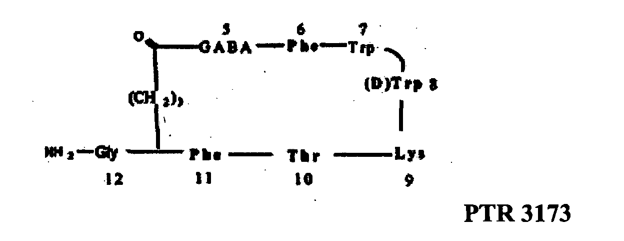

Selon une variante préférée de l'invention, les patientes auxquelles sera destiné le médicament à base de somatostatine ou d'analogue agoniste de la somatostatine mentionné précédemment seront des femmes ayant un facteur de risque de ménopause précoce, et notamment des femmes ayant des antécédents familiaux de ménopause précoce. Selon une variante particulière de l'invention, les patientes auxquelles sera destiné le médicament à base de somatostatine ou d'analogue agoniste de la somatostatine mentionné précédemment seront des femmes atteintes d'une micro-délétion du chromosome X ou d'un syndrome de Turner partiel.

Le second intérêt de la découverte évoquée précédemment réside dans l'opportunité de préparer un médicament à base d'un analogue antagoniste de la somatostatine pour accélérer l'entrée en croissance des follicules quiescents. En effet, un couple sur six parmi ceux qui souhaitent une grossesse présente une difficulté à concevoir. Bien que les causes soient multiples, deux types de traitement ont émergé et sont utilisés de façon régulière en médecine humaine pour le traitement de la stérilité. Ces traitements, aussi appelés "Assistance Médicale à la Procréation" (AMP), consistent d'abord à induire la croissance simultanée de plusieurs follicules pré-ovulatoires. Ceci permet d'obtenir plusieurs ovocytes matures, et donc plusieurs embryons, et ainsi d'augmenter les chances de conception. Ceci est réalisé par l'administration d'un ou plusieurs médicaments stimulant la sécrétion hypophysaire des gonadotrophines (FSH et LH), tel qu'un anti-oestrogène (par exemple le citrate de clomiphène ou le tamoxifène) ou un inhibiteur de l'aromatase (par exemple le létrozole, l'anastrazole ou l'exémestane). La croissance simultanée de plusieurs follicules pré-ovulatoires peut aussi être induite par l'administration d'une préparation de FSH humaine (extractive ou recombinante) associée ou non à de la LH. Lorsque les follicules ont atteint une taille pré-ovulatoire, selon la cause de la stérilité, deux options de traitement existent. La première est de réaliser une insémination intra-utérine (IIU) et la seconde est de prélever les ovocytes dans l'ovaire par aspiration des follicules (entre 5 et 15 ovocytes) et de réaliser une insémination au laboratoire (*in vitro*), soit par simple co-incubation des ovocytes avec les spermatozoïdes du partenaire (FIV) ou par micro-injection d'un spermatozoïde directement dans l'ovocyte (ICSI). L'obtention de plusieurs ovocytes matures est critique pour optimiser les taux de succès (taux de grossesse) obtenus par ces traitements ; or chez certaines femmes, malgré une stimulation ovarienne appropriée, le nombre d'ovocytes obtenus est faible voire même égal à un. Cette difficulté à répondre au traitement stimulant résulte du nombre restreint de follicules en croissance présents dans les ovaires de ces patientes. Il est donc d'un intérêt thérapeutique important de pouvoir mobiliser des follicules de la réserve ovarienne et de les faire entrer en phase de croissance.

Un autre objet de la présente invention consiste en l'utilisation d'un analogue antagoniste de la somatostatine pour préparer un médicament destiné à accélérer l'entrée en croissance des follicules quiescents chez la femme non ménopausée.

L'administration d'un tel médicament pendant une période de 1 à 12 mois chez la femme conduira à une augmentation du nombre de follicules en phase de croissance et donc susceptibles d'être stimulés par les traitements standard pour atteindre le stade de follicules pré-ovulatoires.

L'analogue antagoniste de la somatostatine peut être un polypeptide cyclique ou non cyclique, une protéine de fusion ou de recombinaison, une entité chimique non peptidique (i.e. peptidomimétique) ou encore un peptide *« SS-like »* tel que la corticostatine. Les analogues antagonistes à utiliser devront présenter une haute affinité pour le récepteur SST et inhiber l'activité fonctionnelle de SST14 ou SST28 telle que l'inhibition de la sécrétion d'hormone de croissance par des cellules somatotropes de l'hypophyse et/ou l'inhibition de la prolifération in vitro de cellules d'adénome hypophysaire. De préférence, l'analogue antagoniste de la somatostatine aura une haute affinité pour l'ensemble des sous types de récepteurs de la SST ou alors une affinité privilégiée pour au moins l'un des sous-types 1, 2, 3, 4 et 5.

Un analogue antagoniste de la somatostatine utilisable pour préparer selon l'invention pourra par exemple être un peptide de formule générale

A¹-cyclo{D-Cys-A²-D- Trp-A³ -A⁴-Cys}-A⁵-Y¹ **(I)**

dans laquelle :
A¹ est un α-aminoacide aromatique éventuellement substitué ;
A² est un α-aminoacide aromatique éventuellement substitué ;
A³ est Dab, Dap, Lys ou Orn ;
A⁴ est β-Hydroxyvaline, Ser, Hser, ou Thr ;
A⁵ est un D- ou L- α-aminoacide aromatique éventuellement substitué ; et
Y¹ est OH, NH₂ ou NHR¹, R¹ étant (C₁₋₆)alkyle ;
chaque α-aminoacide aromatique éventuellement substitué étant éventuellement substitué avec un ou des substituants choisis indépendamment parmi le groupe composé d'un atome halogène et des groupes NO₂, OH, CN, (C₁₋₆)alkyl, (C₂₋₆)alkényle, (C₂₋₆)alkynyle, (C₁₋₆)alkoxy, Bzl, O-Bzl et NR⁹R¹⁰, R⁹ et R¹⁰ étant chacun indépendamment H, O, ou (C₁₋₆) alkyle; et
chaque atome d'azote de liaison amide peptidique et le groupe amino de A¹ étant éventuellement substitué avec un groupe méthyle, étant entendu qu'il y a au moins un tel groupe méthyle dans un peptide de formule générale **(I)** ;
ou un sel pharmaceutiquement acceptable d'un peptide de formule générale **(I).**

Par "α-aminoacide aromatique". on entend un résidu aminoacide de formule 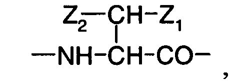 dans laquelle Z₁ est un radical contenant un cycle aromatique et Z₂ est un atome d'hydrogène ou un radical contenant un cycle aromatique. Des exemples de tels radicaux contenant un cycle aromatique incluent, mais ne sont pas limités à, un cycle benzénique ou pyridine et les structures suivantes avec ou sans un ou des substituants X sur le cycle aromatique (X étant, indépendamment à chaque fois qu'il intervient, un atome halogène, NO₂, CH₃, OCH₃, CF₃ ou OH) : 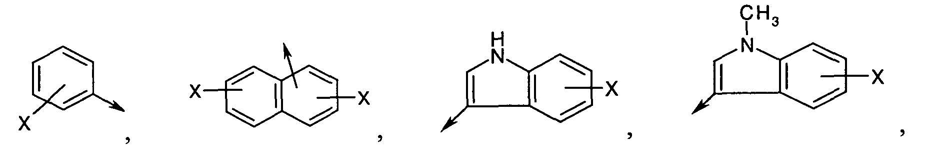 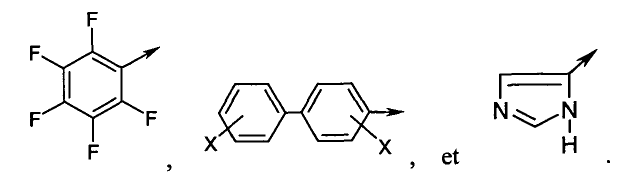 D'autres exemples d'un "α-aminoacide aromatique" selon l'invention sont des His substituées, telles que MeHis, His (τ-Me) ou His (π-Me).

D'autres analogues antagonistes de la somatostatine ont été décrits notamment dans les demandes de brevet PCT WO 98/08528, WO 98/08529, WO 98/24807, WO 98/44921, WO 98/44922, WO 98/45285 et WO 99/22735, ou encore dans les brevets US 6,387,932, US 6,262,229, US 6,063,796, US 6,057,338, US 6,025,372, US 5,925,618, US 5,846,934 et US 4,508,711.

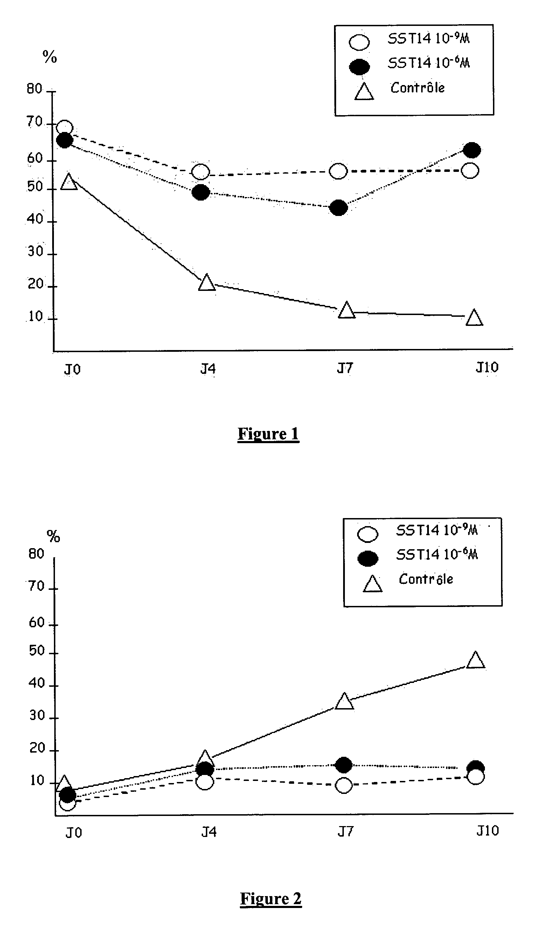

Parmi les analogues antagonistes de la somatostatine utilisables selon l'invention et leurs sels pharmaceutiquement acceptables, on pourra citer plus particulièrement :
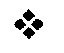 les peptides de formule générale **(I)** suivants :
   - Cpa-cyclo[D-Cys-Pal-D-Trp-N-Me-Lys-Thr-Cys]-D-Trp-NH₂ ;
   - Cpa-cyclo[D-Cys-Tyr-D-Trp- N-Me-Lys-Thr-Cys]-Nal-NH₂;
   - Cpa-cyclo[D-Cys-Pal-D-Trp- N-Me-Lys-Thr-Cys]-Nal-NH₂ ;
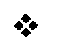 le peptide connu sous le nom de code AC-178,335 (de structure acétyl-D-His-D-Phe-D-Ile-D-Arg-D-Trp-D-Phe-NH₂);
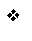 l'octapeptide connu sous le nom de code ODN-8 (cf. Fig. 1 de *Proc. Natl. Acad. Sci. USA* (2000), **97**(25), 13973-13978);
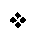 le peptide connu sous le nom de code SB-710411 (de structure Cpa-cyclo[D-Cys-Pal-D-Trp-Lys-Val-Cys]-Cpa-amide) ;
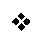 le peptide connu sous le nom de code BIM-23056 (de structure représentée ci-après) ;
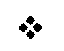 le composé connu sous le nom de code BN-81674 (de structure représentée ci-après) ;
·:· le composé connu sous le nom de code SRA-880 (de structure représentée ci-après) ;
et les sels pharmaceutiquement acceptables de ces derniers.

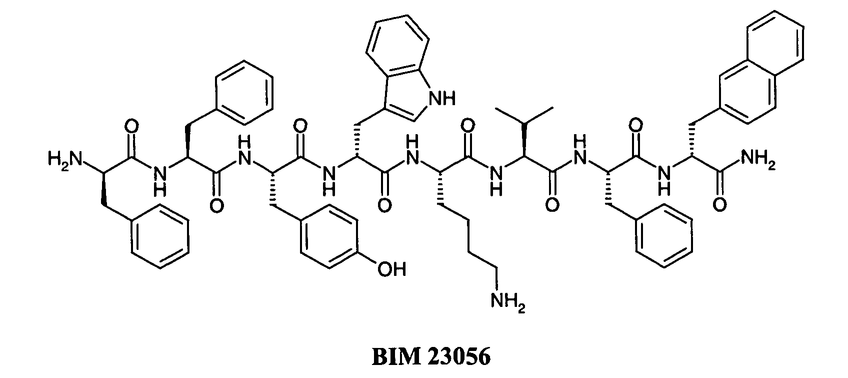

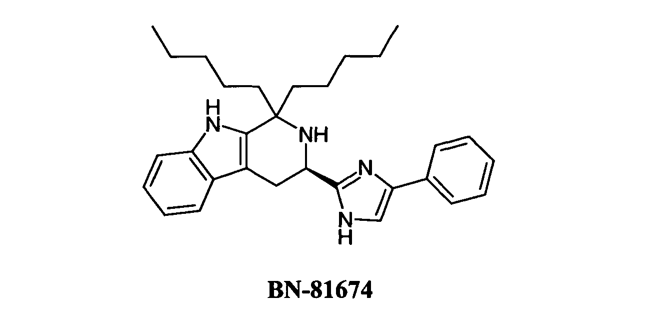

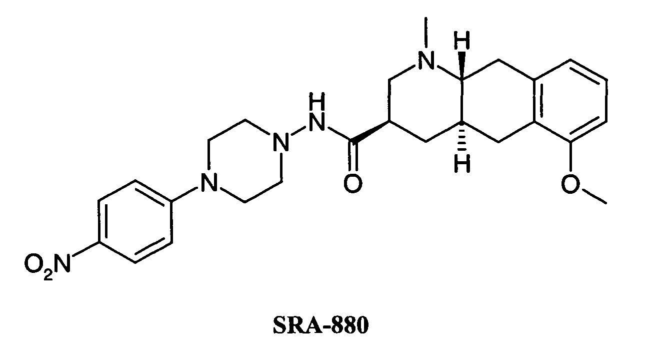

Par analogue agoniste de la somatostatine, on entend un composé pour lequel la dose effective DE₅₀ déterminée au test de l'effet agoniste décrit plus loin est inférieure ou égale à 1 µM pour au moins l'un des sous-récepteurs de la somatostatine.

Par analogue antagoniste de la somatostatine, on entend un composé pour lequel la dose effective DE₅₀ déterminée au test de l'effet antagoniste décrit plus loin est inférieure ou égale à 1 µM pour au moins l'un des sous-récepteurs de la somatostatine.

Par sel pharmaceutiquement acceptable, on entend notamment dans la présente demande des sels d'addition avec des acides inorganiques tels que chlorhydrate, bromhydrate, iodhydrate, sulfate, phosphate, diphosphate et nitrate ou avec des acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", *Int. J. Pharm.* (1986), **33**, 201-217.

Selon la présente invention, les préparations pharmaceutiques contenant de la somatostatine ou un de ses analogues agonistes ou antagonistes applicables à cette invention pourront être administrée par voie parentérale (sous cutanée, intra-musculaire, intra-péritonéale, intra-veineuse, ou en implant), par voie orale, vaginale, rectale, nasale, sublinguale ou transdermale. La voie vaginale sera préférée car elle permet de délivrer des concentrations effectives du principe actif au niveau ovarien tout en minimisant l'exposition systémique. La somatostatine ou l'analogue de la somatostatine utilisé sera formulé avec les excipients nécessaires et connus de l'homme de l'art, pour permettre une administration efficace et reproductible pour chaque voie d'administration.

La dose d'un produit selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

Les situations typiques suivantes pour une utilisation selon l'invention pourraient toutefois être envisagées :
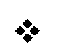 Une patiente d'environ 20 à 25 ans (par exemple) présente un syndrome de Turner partiel par micro-délétion d'un chromosome X. Sa fonction ovarienne est apparemment normale avec des cycles ovulatoires réguliers. Son taux sérique de FSH est légèrement élevé durant la période de transition lutéo-folliculaire (par exemple FSH = environ 9,2 UI/litre). L'échographie ovarienne réalisée par voie trans-vaginale montre des ovaires de volume normaux avec un nombre de follicules antraux légèrement diminué. Considérant son risque élevé de présenter une ménopause précoce, la patiente est traitée avec de l'acétate de lanréotide à une dose de 120 mg/mois (Somatuline® Autogel® 120 mg, Beaufour Ipsen Pharma, France). Le traitement est interrompu après plusieurs années lorsque la patiente souhaite concevoir.
·:· Une patiente d'environ 35 à 40 ans présente une stérilité primaire depuis plusieurs années. Le bilan du couple conclu en une stérilité d'origine tubaire, résultant très probablement d'un antécédent de péritonite. Les cycles menstruels sont ovulatoires et le taux sérique de FSH est légèrement élevé durant la période de transition lutéo-folliculaire (par exemple FSH = environ 11,4 UI/L). L'échographie ovarienne réalisée par voie trans-vaginale montre des ovaires de volume légèrement diminué avec un nombre de follicules antraux diminués (environ 3 par ovaire). Un diagnostic de diminution de la réserve ovarienne est posé. Un traitement par fécondation *in vitro* est recommandé et la patiente entreprend un traitement de stimulation ovarienne par injection quotidienne de 225 unités de FSH recombinante. Au 6^{e} jour de stimulation, une échographie ovarienne montre un seul follicule en croissance de 14 mm dans l'ovaire droit. La dose de FSH est doublée et la patiente est revue 2 jours plus tard. Un seul follicule de 18 mm est observé, confirmant une diminution de la réserve ovarienne. Le traitement est interrompu. Après retour d'un cycle spontané, un traitement par administration quotidienne d'un analogue antagoniste de la somatostatine est initié. Durant ce traitement, le nombre de follicules antraux présents dans chaque ovaire est estimé par échographie en début de chaque cycle menstruel. Après 4 mois de traitement, le nombre de follicules antraux est en moyenne d'environ 6 par ovaire et la FSH sérique a diminué. Une stimulation par FSH recombinante est initiée, un développement folliculaire multiple est obtenu, et une procédure de fécondation *in vitro* classique est effectuée.

### Abréviations et définitions particulières utilisées dans la présente demande :

Les abréviations des acides aminés courants sont en accord avec les recommandations IUPAC-IUB. Par ailleurs, les définitions pour certaines abréviations utilisées dans la présente demande sont comme suit :

| | |
|---|---|
| Abu = | acide α-aminobutyrique ; |
| Aib = | acide α-aminoisobutyrique ; |
| β-Ala = | β-alanine ; |
| Amp = | 4-amino-phénylalanine ; |
| Ava = | acide 5-aminovalérique; |
| Cha = | cyclohexylalanine ; |
| Cpa = | 3-(4-chlorophényl)alanine; |
| Dab = | acide 2,4-diaminobutyrique ; |
| Dap = | acide 2,3-diaminopropionique ; |
| Dip = | 3,3'-diphénylalanine ; |
| GABA = | acide γ-aminobutyrique ; |
| HSer = | homosérine ; |
| 1-Nal = | 3-(1-naphthyl)alanine ; |
| 2-Nal = | 3-(2-naphthyl)alanine ; |
| Nle = | norleucine ; |
| Nva = | norvaline ; |
| 2-Pal = | 3-(2-pyridyl)alanine ; |
| 3-Pal = | 3-(3-pyridyl)alanine ; |
| 4-Pal = | 3-(4-pyridyl)alanine ; |
| Tfm = | trifluorométhyle ; et |
| TfmA = | 4-trifluorométhylphényl-alanine. |

Enfin, Tyr(I) représente un résidu tyrosine iodé (par exemple 3-1-Tyr, 5-1-Tyr, 3,5-I-Tyr) dans lequel l'atome d'iode peut être un isotope radioactif, par exemple I₁₂₅, I₁₂₇ ou I₁₃₁.

Par ailleurs, le terme "environ" fait référence à un intervalle autour de la valeur considérée. Tel qu'utilisé dans la présente demande, "environ X" signifie un intervalle de X moins 10% de X à X plus 10% de X, et de préférence un intervalle de X moins 5% de X à X plus 5% de X.

### Préparation des peptides de formule générale (I) :

Les peptides de formule générale **(I)** mentionnés ci-dessus et leur synthèse sont décrits dans la demande de brevet PCT WO 02/072602.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

L'exemple suivant est présenté pour illustrer les procédures ci-dessus et ne doit en aucun cas être considéré comme une limite à la portée de l'invention.

### EXEMPLE

Des ovaires de brebis adultes sont collectés immédiatement après abattage. Les ovaires sont placés dans un milieu de transport d'organes sans sérum (X-vivo, Bio Whittaker, Walkersville, MD, USA) à 10 °C et acheminé au laboratoire. Environ 1 h après le prélèvement, le cortex est isolé de la medulla puis fractionné en tranches de 2 mm d'épaisseur (1 cm², poids moyen de 212 mg) après rinçage dans du X-vivo neuf. Les fragments de cortex sont cultivés en étuve sous 5% d'oxygène pendant 10 jours dans des boites à puits en présence de DMEM. Le milieu est changé tous les 2 jours.

Dans les fragments contrôles (incubées en l'absence de SST) les follicules primordiaux passent progressivement vers le stade de follicule en début de croissance (voir les Figures 1 et 2). L'addition de SST14 à des concentrations variant entre 10⁻⁹ M et 10⁻⁶ M inhibe très significativement l'entrée des follicules primordiaux en croissance comme le montre le maintien au cours du temps du nombre de follicules primordiaux (cf. Figure 1) et l'absence d'augmentation du nombre de follicules primaires (cf. Figure 2).

### Brève description des figures

La **Figure 1** représente les proportions de follicules au repos au cours d'une période de 10 jours de culture de cortex ovarien de brebis. Ces proportions sont mesurées pour chaque échantillon testé au jour du début de l'expérience (J0), au 4^{e} jour (J4), au 7^{e} jour (J7) et au 10^{e} jour (J10).

La **Figure 2** représente les proportions de follicules primaires au cours d'une période de 10 jours de culture de cortex ovarien de brebis. Ces proportions sont mesurées pour chaque échantillon testé au jour du début de l'expérience (J0), au 4^{e} jour (J4), au 7^{e} jour (J7) et au 10^{e} jour (J10).

### Tests de détermination de l'effet agoniste ou antagoniste d'un analogue de la somatostatine

### Inhibition de la production intracellulaire d'AMPc

Des cellules CHO-K1 exprimant les sous-types de récepteurs de la somatostatine humaine (SRIF-14) sont cultivées dans des plaques à 24 puits dans un milieu RPMI 1640 contenant 10% de sérum foetal de veau. Le milieu est changé le jour précédant l'expérience.

Les cellules à raison de 10⁵ cellules/puits sont lavées 2 fois avec 0,5 ml de nouveau milieu RPMI comprenant 0,2 % BSA complété par 0,5 mM de 3-isobutyl-1-méthylxanthine (IBMX) et incubées pendant environ 5 minutes à environ 37 °C. La production d'AMP cyclique est stimulée par l'addition de 1 mM de forskoline (FSK ; fournisseur: Sigma Chemical Co., St. Louis, MO, USA) pendant 15-30 minutes à environ 37 °C.

### Détermination de l'effet agoniste d'un analogue de la somatostatine

L'effet agoniste d'un analogue de la somatostatine est mesuré par l'addition simultanée de FSK (1 µM) et de l'analogue à tester (10⁻¹⁰ M à 10⁻⁵ M).

Le milieu réactionnel est éliminé et 200 ml de HCl 0,1 N sont ajoutés. La quantité d'AMPc est mesurée par un test radioimmunologique (Kit FlashPlate SMP001A, New England Nuclear, Boston, USA).

### Détermination de l'effet antagoniste d'un analogue de la somatostatine

L'effet antagoniste d'un analogue de la somatostatine est mesuré par l'addition simultanée de FSK (1 µM) , SRIF-14 (1 à 10 nM) (fournisseur : Bachem, Torrence, CA, USA) et de l'analogue à tester (10⁻¹⁰ M à 10⁻⁵ M).

Le milieu réactionnel est éliminé et 200 ml de HCl 0,1 N sont ajoutés. La quantité d'AMPc est mesurée par un test radioimmunologique (Kit FlashPlate SMP001A, New England Nuclear, Boston, USA).

## Revendications

1. Utilisation de la somatostatine ou d'un de ses analogues agonistes pour préparer un médicament destiné à réguler la réserve folliculaire ovarienne, et en particulier à diminuer l'épuisement de la réserve folliculaire ovarienne avec le temps, chez la femme non ménopausée.

2. Utilisation selon la revendication 1, **caractérisée en ce que** la somatostatine est utilisée pour préparer le médicament.

3. Utilisation selon la revendication 1, **caractérisée en ce qu'**un analogue agoniste de la somatostatine est utilisé pour préparer le médicament.

4. Utilisation selon la revendication 3, **caractérisée en ce que** l'analogue agoniste de la somatostatine est choisi parmi le groupe composé du lanréotide, de l'octréotide, du vapréotide, du SOM 230, du MK-678, du BIM-23190, du BIM-23197, du BIM-23268, du PTR-3173, du TT-232 et des sels pharmaceutiquement acceptables de ces derniers.

5. Utilisation selon la revendication 4, **caractérisée en ce que** l'analogue agoniste de la somatostatine est le lanréotide ou un de ses sels pharmaceutiquement acceptables de ces derniers.

6. Utilisation selon l'une des revendications 1 à 5, **caractérisée en ce que** le médicament est destiné à être administré à une femme ayant un facteur de risque de ménopause précoce.

7. Utilisation selon l'une des revendications 1 à 5, **caractérisée en ce que** le médicament est destiné à être administré à une femme atteinte d'une micro-délétion du chromosome X.

8. Utilisation d'un analogue antagoniste de la somatostatine pour préparer un médicament destiné à accélérer l'entrée en croissance des follicules quiescents chez la femme non ménopausée.

9. Utilisation selon la revendication 8, **caractérisée en ce que** l'analogue antagoniste de la somatostatine est choisi parmi les peptides de formule générale **(I)**
A¹-cyclo{D-Cys-A²-D-Trp-A³-A⁴-Cys}-A⁵-Y¹ **(I)**
dans laquelle :
A¹ est un α-aminoacide aromatique éventuellement substitué ;
A² est un α-aminoacide aromatique éventuellement substitué ;
A³ est Dab, Dap, Lys ou Orn ;
A⁴ est β-Hydroxyvaline, Ser, Hser, ou Thr ;
A⁵ est un D- ou L- α-aminoacide aromatique éventuellement substitué ; et
Y¹ est OH, NH₂ ou NHR¹, R¹ étant (C₁₋₆)alkyle ;
chaque α-aminoacide aromatique éventuellement substitué étant éventuellement substitué avec un ou des substituants choisis indépendamment parmi le groupe composé d'un atome halogène et des groupes NO₂, OH, CN, (C₁₋₆)alkyl, (C₂₋₆)alkényle, (C₂₋₆)alkynyle, (C₁₋₆)alkoxy, Bzl, O-Bzl et NR⁹R¹⁰, R⁹ et R¹⁰ étant chacun indépendamment H, O, ou (C₁₋₆) alkyle; et
chaque atome d'azote de liaison amide peptidique et le groupe amino de A¹ étant éventuellement substitué avec un groupe méthyle, étant entendu qu'il y a au moins un tel groupe méthyle dans un peptide de formule générale **(I)**;
et les sels pharmaceutiquement acceptables de tels peptides.

10. Utilisation selon la revendication 8, **caractérisée en ce que** l'analogue antagoniste de la somatostatine est choisi parmi le groupe composé :
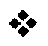 les peptides suivants :
- Cpa-cyclo[D-Cys-Pal-D-Trp-N-Me-Lys-Thr-Cys]-D-Trp-NH₂ ;
- Cpa-cyclo[D-Cys-Tyr-D-Trp- N-Me-Lys-Thr-Cys]-Nal-NH₂ ;
- Cpa-cyclo[D-Cys-Pal-D-Trp- N-Me-Lys-Thr-Cys]-Nal-NH₂ ;
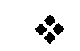 le peptide connu sous le nom de code AC-178,335 ;
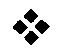 l'octapeptide connu sous le nom de code ODN-8 ;
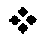 le peptide connu sous le nom de code SB-710411 ;
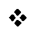 le peptide connu sous le nom de code BIM-23056 ;
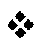 le composé connu sous le nom de code BN-81674 ;
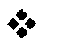 le composé connu sous le nom de code SRA-880 ;
et des sels pharmaceutiquement acceptables de ces derniers.
